# EUROPEAN PATENT APPLICATION

(11) **EP 4 785 994 A1**
(43) Date of publication of application: **05.08.2026**
(21) Application number: 25155207.1
(22) Date of filing: 31.01.2025
(51) Int. Cl.: A61N 5/10, A61B 5/055

(54) **METHOD FOR DETERMINING AN EFFECTIVE DOSE EMITTED BY RADIOEMBOLIZATION PARTICLES**

(71) Applicant: Siemens Healthineers AG, 91301 Forchheim (DE)
(72) Inventor: Georgi, Jens-Christoph, 90522 Oberasbach (DE); Preuhs, Alexander, 91058 Erlangen (DE); Regensburger, Alois, 91099 Poxdorf (DE); Tashenov, Stanislav, 91336 Heroldsbach (DE)
(74) Representative: Siemens Healthineers Patent Attorneys

(57) **Abstract**

The invention relates to a computer-implemented method for determining an effective dose emitted by radioembolization particles, the method comprising:
- obtaining a medical image including at least one target area of a patient, the target area including a plurality of image-detectable radioembolization particles;
- determining, by means of an image analysis of the medical image, at least one particle distribution information, the particle distribution information indicating a distribution of the radioembolization particles in the target area;
- determining an effective dose emitted by the radioembolization particles, by applying a non-proportional distribution-to-dose mapping to the determined at least one particle distribution information.

## Description

Independent of the grammatical term usage, individuals with male, female or other gender identities are included within the term.

The present invention is related to methods for determining an effective dose emitted by radioembolization particles. The invention is further related to a corresponding data processing apparatus, computer program product and computer-readable storage medium.

Radioembolization can be used to treat various conditions such as cancers or other abnormal tissue growth of a target tissue. In particular, radioembolization can be performed to treat abnormal tissue growth in the liver of a patient. Preferably, radioembolization incorporates both, embolization as well as radiation therapies.

Radioembolization typically includes the introduction of particles into the blood stream of an anatomical structure, in particular a blood vessel of a patient, wherein the anatomical structure is positioned at or near a target tissue (e.g., a tumor). Primarily, the particles may include a radioactive isotope that delivers a dose of radiation to the target tissue. Optionally, the particles may at least partially occlude the blood vessels at the target tissue reducing or preventing blood flow to the target tissue. Alternatively, the particles may be distributed in the blood vessels without occluding the vessel. The dose of radiation to the target tissue in combination with the optional reduction and/or prevention of blood flow to the target tissue may destroy the target tissue, shrink the size of the target tissue, and/or reduce growth of the target tissue.

However, existing radioembolization methods suffer from various drawbacks. Existing radioembolization particles are difficult to position in a desired location. In addition, it can be difficult to determine a dose of radiation that is delivered to the target tissue. Thus, radioembolization particles may be positioned in sub-optimal and/or unknown locations during a treatment procedure and may deliver unknown radiation doses.

In particular, the radioembolization particles tend to cluster in vessels or other anatomical spaces after they have been injected and are not homogeneously distributed. In these dense clusters of radioembolization particles, significant amounts of self-absorption or cross-absorption will occur by the surrounding particles, and a significant fraction of the radioactive emission will not reach the surrounding tissue. Therefore, the estimation of the effective dose, which is delivered to the surrounding tissue, in particular to the target tissue, but also to surrounding healthy tissue, is unprecise. Such drawbacks may lead to ineffective treatments, sub-optimal treatments, and/or to unnecessarily high radiation doses being delivered to healthy tissues.

There exists a need, therefore, for improving the estimation of an effective dose delivered by radioembolization particles. In particular, the estimation of delivered effective dose shall be more precise. In particular, the estimation shall be applicable to different anatomic structures and shall be executable in real-time or close to real-time, i.e. within an acceptable amount of time, even with limited computational resources.

This task is solved by the features of the independent claims. Further advantageous examples are included in the dependent claims.

According to an aspect, the invention relates to a computer-implemented method for determining an effective dose emitted by radioembolization particles. The method may comprise the following steps:
- obtaining a medical image including at least one target area of a patient, the target area including a plurality of radioembolization particles, wherein at least a part of the radioembolization particles is image-detectable;
- determining, by means of an image analysis of the medical image, at least one particle distribution information, the particle distribution information indicating a distribution of the radioembolization particles in the target area;
- determining an effective dose emitted by the radioembolization particles, by applying a non-proportional distribution-to-dose mapping to the determined at least one particle distribution information.

One important application in this connection is targeted radioembolization of blood vessels, for example, of blood vessels supplying a tumor. In this case, a radioembolization agent including a plurality of radioembolization particles is administered. The radioembolization particles accumulate in a particular portion of at least one blood vessel. Preferably, at least some of the radioembolization particles include at least one radioactive ingredient, for example for conducting selective internal radiation therapy (SIRT). After the administration, the radioembolization particles may cluster and optionally occlude the vessel at least partially.

Alternatively, a cluster of radioembolization particles may not have a significant effect on blood flow through the vessel.

A primary field of application of such radioembolization methods is the treatment of advanced-stage liver cancer. Other applications have been reported for example for radioembolization of glioblastoma in the brain. In an exemplary embodiment, the radioembolization particles are microparticles, e.g., microbeads, loaded with the radioactive ingredient.

The success of therapy greatly depends on the accuracy of the embolization. In particular, the target tissue to be treated, in particular a tumor, should be embolized with an adequate quantity of the radiation. In other words, the effective dose emitted by the embolization particles, which is delivered to the target should be at an adequate magnitude. On the other hand, healthy tissue should be spared as far as possible. In other words, the effective dose emitted by the embolization particles to healthy tissue should be kept as low a possible.

In some cases, highly selective radioembolization takes place with high local doses in order to achieve optimum therapy success. For example, in the case of SIRT, highly selective radioembolization with a dose of 100 Gy per lesion in the case of liver cancer is reported to increase the survival time from six months to 14 months. Such high doses may only be selectively administered in order to spare healthy tissue. Thus, monitoring of the embolized volume is important with selective radioembolization.

In the present disclosure, the term "effective dose" is used for the amount of dose or radiation emitted by the radioembolization particles which is actually absorbed by the surrounding tissue or the tumor to be treated. In some examples, the "effective dose" is the "emitted dose" minus the "shielded/absorbed dose", wherein the "shielded/absorbed dose" is the quantity of dose absorbed by the surrounding radioembolization particles. In particular, if the radioembolization particles are included in a blood vessel, an effective dose may be the dose emitted to surrounding tissue. The ratio of shielded/absorbed dose and effective dose may be directionally and spatially inhomogeneous depending on the specific clustering arrangements of the particles.

A medical image may be or may include a two-dimensional image. The medical image may be or may include a three-dimensional image. The medical image may be or may include a four-dimensional image, where there are three spatial and one time-like dimensions. The medical image may comprise a plurality of individual medical images and/or a plurality of slices.

The medical image comprises image data, for example, in the form of image points, i.e., a two-or three-dimensional array of pixels or voxels. Such arrays of pixels or voxels may be representative of color, intensity, absorption or other parameters as a function of two or three-dimensional position, and may, for example, be obtained by suitable processing of measurement signals obtained by a medical imaging modality or image scanning facility.

A medical image may be a radiology image depicting a body part of a patient. Accordingly, it may contain two or three-dimensional images of the patient's body part. The medical image may be representative of an image volume or a cross-section through the image volume. The patient's body part may be comprised in the image volume.

A medical imaging modality corresponds to a system used to generate or produce medical image data. For example, a medical imaging modality may be a computed tomography system (CT system), a magnetic resonance system (MR system), an angiography (or C-arm X-ray) system with optional CBCT (cone-beam CT) capability, a positron-emission tomography system (PET system) or the like. Specifically, computed tomography is a widely used imaging method and makes use of "hard" X-rays produced and detected by a spatially rotating instrument. The resulting attenuation data (also referred to as raw data) is processed by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas and the signals are analyzed by a computer creating detailed images of the internal structures in any section of the human body.

Accordingly, the depicted body part of the patient in general will comprise a plurality of anatomical structures, e.g., blood vessels, and/or organs.

The medical image may comprise a plurality of images or image slices. The slices respectively show a cross-sectional view of the image volume. The slices may comprise a two-dimensional array of pixels or voxels as image data. The arrangement of slices in the medical image may be determined by the imaging modality or by any post-processing scheme used. Further, slices may artificially be defined in the imaging volume spanned by the medical image. Optionally, this may happen as a function of the image data comprised in the medical image in order to optimally pre-process the medical image for the ensuing diagnostic workflow.

The medical image data may be stored in a standard image format such as the Digital Imaging and Communications in Medicine (DICOM) format and in a memory or computer storage system such as a Picture Archiving and Communication System (PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

In the present disclosure, the term "target area" shall be understood broadly and shall refer to an area of the human body comprising radioembolization particles. In particular, the target area may be or may include an anatomical structure, in particular a blood vessel, of the patient. The target area may include a tumor. The target area may include surrounding tissue. Preferably, the surrounding tissue may be tissue surrounding the anatomical structure and/or a blood vessel. Preferably, the surrounding tissue shall not be subject to high effective dose.

Preferably, at least some of the plurality of radioembolization particles in the target area may be image-detectable. This means, that in an image, in particular in a medical image, these particles show a contrast which deviates from the contrast of surrounding tissue. In other words, when an imaging procedure is conducted, the particles may behave differently than the surrounding tissue. In particular, the particles may show a different x-ray absorption than the surrounding tissue. In particular, the particles may show a different magnetic resonance than the surrounding tissue. The radioembolization particles are preferably detectable in medical images, in particular radiological images. The particles may be detectable individually, and/or their concentration or density may be determined in a region of interest. In particular, the particles may be image detectable when forming a radioembolization particle micro cluster. In other words, it is not required that the particles are image detectable individually. By contrast, the image-detectability of the particles may be given, as said, when the particles are bundled in a micro cluster, such that the properties of the particles add up and/or interact with each other to make the micro cluster as a whole visible in medical images.

Preferably, the radioembolization particles are at least partially radiopaque. Thus, the radioembolization particles and/or micro clusters of radioembolization particles are detectable or visible in X-ray images, CT-images and/or Cone-Beam Computed Tomography (CBCT) images.

Additionally or alternatively, the radioembolization particles may be detectable and/or visible in an MRI image. Additionally or alternatively, the radioembolization particles may be detectable and/or visible in an ultrasound image.

According to an example, the plurality of radioembolization particles may include radioembolization particles, which are image-detectable, and radioembolization particles, which are not image-detectable. As a part of the radioembolization particles is image-detectable, the plurality of radioembolization particles in its entirety may image detectable. In particular, the image-detectable radioembolization particles may be mixed with the non-image-detectable radioembolization particles. In particular the image-detectable radioembolization particles may from a radioembolization particle micro cluster in combination with the non-image-detectable radioembolization particles.

Due to the image-detectability of the radioembolization particles, a particle distribution information can be determined based on the medical image. The particle distribution information may be any information suitable for indicating a distribution of the radioembolization particles in the target area.

In particular, the particle distribution information may be a density based information. Density based information may also be considered to be concentration based information. The particle distribution information may include a information of the density or concentration of the radioembolization particles in a specific region of the target area, e.g., the particle distribution information may include information about the density of radioembolization particles in an image point, in particular a specific pixel or voxel of the medical image.

In addition or as an alternative, and as further described below, the particle distribution information may include information about the clustering behavior of radioembolization particles, in particular on a clustering shape, clustering size and/or clustering size.

The particle distribution information may allow conclusions, whether the radioembolization particles are located in desired positions. The desired positions in a target tissue may be determined prior to the treatment using a diagnostic or other procedure that may analyze the vasculature of the target tissue. The desired positions may correspond to the locations of blood supply to the target tissue. The desired positions may correspond to multiple locations of blood supply so that the radiation is delivered to target tissue. The desired positions may correspond to regions of a tumor with altered vascularization.

In the framework of the present disclosure, image analysis is to be understood broadly and refers to the process of processing a medical image to determine particle distribution information. In particular, the image analysis may include generating a particle density map. A particle density map may describe the density/concentration of radioembolization particles in the medical image.

The distribution-to-dose mapping may map one or more specific distribution information, e.g., a particle density or a cluster shape, to a specific dose value. Thus, the distribution-to-dose mapping represents the relationship between the distribution information and the resulting level of effective dose. The distribution-to-dose mapping is non-proportional, in particular sub-proportional. The term "proportional" shall be interpreted in the sense of "directly proportional". Thus, a mapping, here the mapping between the distribution information and the effective dose, may be named proportional or directly proportional if their corresponding elements have a constant ratio.

Thus, self-absorption or cross-absorption between the radioembolization particles in the target area is taken into account. In particular for high particle densities, the resulting level of determined effective dose can be lower than the effective dose level which would result from a proportional or linear distribution-to-dose mapping. In the present disclosure, the terms "proportional" and "linear" are used interchangeably.

The non-proportional distribution-to-dose mapping may include at least one non-linear, in particular sub-linear distribution-to-dose translation curve. In particular, the non-linear distribution-to-dose translation curves may map a specific particle density, determined based on the medical image, to a specific effective dose value. Alternatively or in addition, the non-proportional distribution-to-dose mapping may include multiple translation curves, in particular clustering-type-specific translation curves. Each of the clustering-type-specific translation curves may map a particle density of a particular clustering type to a specific effective dose value. Each of the clustering-type-specific translation curves may be proportional or non-proportional. In any case, in this embodiment, the non-proportionality of the distribution-to-dose mapping comes from the possibility of discrete switching between the multiple available translation curves. The switching between the multiple translation curves may be conducted depending on a determined clustering-type of a specific particle cluster. In one embodiment, a statistical mixture of several clustering types may be assumed. Thus, one or multiple of the translation curves may represent a statistical mixture of the respective translation curves of the respective clustering types.

Optionally, a dose map may be generated by means of the image analysis and the non-proportional distribution-to-dose mapping. A dose map may describe the radiation dose delivered to the target based on the location and distribution of the visible radiopaque radioembolization particles.

Thus, by taking into account self-shielding effects resulting from clustering of radioembolization particles, in particular of radioactive SIRT radioembolization particles, the effective dose can be determined more accurately and more precisely. Due to the, preferably pre-established, distribution-to-dose mapping, the determination of an effective dose-level can be conducted in real time or close to real-time, even during a medical procedure. In other words, the determination of a dose level is executable within an acceptable amount of processing time. Acceptable processing times may be less than a second, less than 10 seconds, less than a minute, or less than 10 minutes, e.g.

In a preferred embodiment, the radioembolization particles include radiopaque material. The radiopaque material may be radioactive material. In particular, the radiopaque material and the radioactive material may be at least partly the same material. This means, that radioactive material may contribute to radiopaque property of the radioembolization particles. In other words, the radioembolization particles may include radioactive material, which is itself radiopaque to a certain degree. Optionally, the radioembolization particles may include additional material without radioactive but with radiopaque properties, at least to a certain degree. Optionally, the radioembolization particles may include radioactive material which is weakly radiopaque. According to an example, the radioembolization particles may be used in a selective internal radiation therapy (SIRT) treatment. The ratio of radiopaque and radioactive material may influence the imaging characteristics of the radioembolization particles.

As explained before, another way to adjust or influence the imaging characteristics of the plurality of radioembolization particles may be to mix radioembolization particles, which are image-detectable, with radioembolization particles, which are not image-detectable.

In various embodiments of the present disclosure, radiopaque glass radioembolization particles are provided. The radiopaque glass radioembolization particles can be used for radioembolization treatments to treat abnormal tissues in a patient. In an example radioembolization treatment, the radioembolization particles of the present disclosure may be injected into the blood stream of a patient and directed to a target tissue (e.g., a tumor). The radioembolization particles can deliver a dose of radiation to the target tissue. Optionally, the radioembolization particles may stop and/or may reduce the blood supply to the target tissue, but this is not required. The radioembolization treatment can destroy the target tissue, reduce the size of the target tissue, and/or limit growth of the target tissue.

Preferably, at least a fraction of the radioembolization particles is at least partially radiopaque. The term radiopaque is used in the present disclosure to describe a property of the particles that makes the particles opaque to various forms of radiation such as x-rays. With this property, the radioembolization particles of are visible in 2D and 3D x-ray images and in computed tomography (CT) images, in particular CBCT images. In other words, concentration and absorption properties of radioembolization particles at X-ray imaging wavelength are sufficient to produce a detectable contrast in a reconstructed X-ray image.

Standard radioembolization particles are only very weakly radiopaque. Thus, the standard radioembolization particles are typically not visible in x-ray images, when the radioembolization particles are provided in the standard therapeutic concentrations/densities used for medical treatment. Nevertheless, x-ray images are typically used in a clinical setting (e.g., an operating room) where a radioembolization treatment is performed. Thus, standard treatment methods may include post-treatment imaging in which a location of the radioembolization particles can be determining using single photon emission computed tomography (SPECT) imaging devices and/or positron emission tomography (PET) imaging devices or other post-treatment devices. Such posttreatment devices require the patient to be moved from the clinical setting (e.g., operating room) to another location to perform such image capture. This requirement does not allow a medical professional to determine a location of the radioembolization particles during treatment so that corrective or remedial actions can be taken in real-time or close to real-time.

Thus, using radiopaque, in particular highly or sufficiently radiopaque, radioembolization particles is an improvement over standard particles and treatment methods, as it allows imaging to be performed in the clinical setting without the need to move the patient from the operating room (cite state of the art here). X-ray devices and/or beam CT scan devices can be used in the clinical setting to provide information to the medical professional regarding a location of the radioembolization particles. By combining this with the more precise and more accurate method for determining an effective dose, the treatment of patients can be improved. In particular, a reliable determination of effective dose, e.g., by means of dose maps, can be achieved in real-time so that the treatment can be adjusted while the patient is in the clinical setting. These improvements can result in improved effectiveness of the treatment and reduced likelihood that healthy tissues are unnecessarily harmed during treatment.

In an exemplary embodiment, the radioembolization particles may include at least one of a radioisotope, in particular Y90 and/or Ho166. In particular, the radioembolization particles may include radioactively activated Yttrium, Silicon. The radiopaque properties of the radioactive material, in particular Yttrium, Silicon, may be relatively weak in some embodiments, whereas in other embodiments, the radiopaque properties of the radioactive material may be particularly high. Thus, the radioembolization particles may include additional/further radiopaque material. In particular, only a part of the material of the radioembolization particles may be radioactively activated, wherein the remaining part of the material of the radioembolization particles may be not activated. The additional radiopaque material can be a suitable material with a strong tendency to block x-ray radiation so that the radioembolization particle is more visible in an x-ray based image. In various examples, the radiopaque material may include at least one of Holmium, Samarium, Iodine, Barium, Tantalum, Iridium, Rhenium, or Indium. The materials of the radioembolization particles may be mixed and/or may be arranged in layers and/or one of the materials may be provided as a coating to the other material. As explained above, the radioactive material, as for example activated Yttrium, may by itself contribute to radiopacity of the radioembolization particle.

In a preferred embodiment, the medical image includes a plurality of target areas, and the method comprises:
- determining particle distribution information for each of the target areas; and
- determining an effective dose emitted by the radioembolization particles, by applying a non-proportional distribution-to-dose mapping to the determined respective particle distribution information. In other words, the method may include a loop over multiple target areas of a medical image and determining a respective particle distribution and effective dose based on that. The respective effective dose values, which are determined for the respective target areas may influence each other. Thus, an effective dose value for a target area may be calculated based on other effective dose values calculated for other target areas in the medical image. In consequence, the determined effective dose value may be more precise.

Further, a cumulative effective dose value may be calculated based on the effective dose values determined for several or all of the target areas. In particular, the cumulative effective does may be determined by merging the multiple effective dose values, e.g., by adding the multiple effective dose values. The cumulative effective dose value may describe the total effective dose emitted to an anatomical structure or a tissue covering multiple target areas.

Preferably, the step of determining at least one particle distribution information includes:
- determining, based on the medical image, a particle density map, wherein a value of image points, in particular of pixels or voxels, of the particle density map correlates with the density of radioembolization particles in the corresponding image points of the medical image.

In particular, a determination of the density of radioembolization particles may be conducted based on the medical image, which may be a CBCT and/or CT and/or MRI image. In particular, the determination may include determining a change of Hounsfield units caused by the radioembolization particles and/or other spatial density measurements of the radioembolization particles. This can e.g. be done via subtraction imaging or spectral imaging methods in case of CT/CBCT. In the case of MRI-imageable radioembolization particles, like Holmium beads, e.g. Quirem by Terumo, the determination may be performed by means of a specific MRI response/signal of the MRI-imageable particles.

If a medical image includes more than target area, the particle density map may be determined based on the interaction of radioembolization particles in various target areas with each other. Thereby, a more precise particle map may be determined.

In a preferred embodiment, the non-proportional distribution-to-dose mapping is based on the dose self-shielding and/or the dose cross-shielding between the radioembolization particles. In particular, the non-proportional distribution-to-dose mapping may be based on the dose self-shielding and/or the dose cross-shielding between the radioembolization particles in a particle micro cluster.

As already briefly explained earlier, normally, the radioembolization particles do not distribute uniformly in an anatomical structure, in particular a vessel, in which the particles are injected. Instead, radioembolization particles tend to cluster or stack inside of the anatomical structure. While this effect is desired on the one hand to allow the particles to fulfill their radioembolization function, on the other hand the precision of effective dose estimation is negatively influenced.

In particular, the dose emitted by particles inside of a cluster or micro cluster, is shielded by the surrounding particles. By contrast, the radioembolization particles forming the outer shell of a micro cluster may be capable of emitting dose that is not passing through other particles before it reaches the surrounding tissue. Thus, due to the shielding, the total emitted dose of an entire micro cluster is lower than the sum of the dose emitted by the individual particles.

However, the inner and outer radioembolization particles will contribute equally to the dose distribution information, e.g. the attenuation observed on a medical image, e.g. a CT or MRI image. Thus, by applying a non-proportional or non-linear, in particular sub-proportional or sub-linear, distribution-to-dose mapping, e.g. for mapping the attenuation in a medical image to an effective dose value, the shielding effects can be taken into account and the estimation of the emitted dose is more precise. Therefore, also the therapeutic effect on the surrounding tissue can be better assessed.

Preferably, the particle distribution information includes an average density of the radioembolization particles in the target area, in particular in an anatomical structure, such as a vessel. The average density of radioembolization particles may be determined based on the attenuation derivable form medical image. In particular, the distribution-to-dose mapping includes a non-proportional, in particular sub-proportional, saturation curve, which is indicative of a correlation between the average particle density, in particular the average particle density of a micro cluster, and the effective dose.

By assuming a saturation-type non-proportional, in particular sub-proportional behavior of particle-density-to-dose-level relationship, the physical shielding properties of the radioembolization particles are taken into account. In particular, when radioembolization particles are used, which are simultaneously radiopaque to be detectable on medical images, the shielding effects need to be taken into account.

Preferably, the distribution-to-dose mapping, in particular the saturation curve, may be predetermined. This allows for a real-time application of the saturation curve to obtained particle distribution information. Example ways to determine the saturation curve may include Monte Carlo simulations of multiple random radioembolization particle distributions and/or of multiple radioembolization particle micro clusters with different average particle densities. The Monte Carlo simulation may include the simulation of self-absorption and/or cross-absorption of a fraction of the emitted dose.

Alternatively or in addition, animal experiments and/or clinical experiments may be conducted, in which the particle distribution, in particular the clustering of particles, may be measured in vivo. A ground truth for the dose emitted by the particles may be established by conducting a PET/CT scan, e.g. The measured particle distribution may then be mapped to the measured emitted dose.

Alternatively or in addition, the effective emitted dose, and/or activity of radioembolization particle clusters may be measured locally by nuclear dose/activity measurement devices. In an exemplary arrangement, the dose/activity measurement devices may be placed around phantoms of anatomical structures with radioembolization particles.

Alternatively or in addition, the statistical clustering behavior of radioembolization particles may be observed in anatomical phantoms. For example, cluster configurations may be studied via microscopic (X-ray or optical) imaging. The self-shielding in an observed simulation, e.g. by means of one of the above-mentioned methods.

In a preferred embodiment, the method comprises: selecting, based on particle distribution information, a target saturation curve from on a plurality of pre-defined saturation curves. As explained earlier, the emission characteristics of the radioembolization particles depend strongly on the clustering. E.g., larger clusters may have different emission characteristics than smaller clusters. Also, within a cluster, the emission characteristics may vary locally. Thus, depending on the clustering type, in particular size, shape etc., a different saturation curve may be more suitable for approximating dose emission while taking into account the self-shielding of the clusters.

In particular, the pre-defined saturation curves may be parametrized differently such that, based on the particle distribution information of a cluster, a corresponding pre-defined saturation curve may be selected.

In an example, each of the saturation curves/the pre-defined saturation curves is or was parametrized based on Monte Carlo Simulation of random particle distributions with a specific average density, size, shape etc., and/or based on experimentally measuring particle distributions and corresponding effective dose values. The pre-definition process of the saturation curves may be conducted before the claimed method is executed. Alternatively, the pre-definition process may be part of a claimed method and/or the claimed method may include a refinement step for refining at least one saturation curve, in particular a set of provided saturation curves, to better fit to a particular application. For the refinement of the saturation curves, similar approaches as for the pre-definition may be applied. The refinement may allow for a more precise determination of the effective emitted dose values.

In a preferred embodiment, the particle distribution information indicates and/or includes the local distribution of radioembolization particles in the target area, in particular in an anatomical structure. In particular the local distribution may include information about a size of radioembolization particle micro clusters and/or a shape of radioembolization particle micro clusters and/or a distribution of radioembolization particle micro clusters, in particular a 3D shape and/or 3D distribution of radioembolization particle micro clusters. The more information is present about the characteristics of radioembolization particle micro clusters, the more accurate can the determination of the effective dose be. Especially the information about the 3D shape of micro clusters and the distribution of the micro clusters in the target area and/or the distribution of micro clusters among multiple target areas may be used for determining the effective dose.

In a preferred embodiment, a clustering type of a radioembolization particle micro cluster is determined by inputting the medical image and/or the particle density map into a machine learning algorithm, in particular a clustering type determination algorithm, wherein the machine learning algorithm, in particular the clustering type determination algorithm, is trained to determine a clustering type in particular based on imaging characteristics of the radioembolization particle micro clusters.

In the framework of the present disclosure, the term "clustering type" is to be understood broadly and refers to a group of radioembolization particle micro clusters with similar characteristics and/or properties. Any characteristics and/or properties of a radioembolization particle micro clusters may be used for defining a clustering type. In particular, the clustering type may be defined based on imaging characteristics of a radioembolization particle micro cluster, such as attenuation which is detectable in a medical image. In particular, the clustering type may be defined based on physical properties of a radioembolization particle micro cluster, such as size, shape, in particular 3D shape, distribution, dimensions etc. In particular, the clustering type may be defined based on the reaction of particle clusters to a specific biological and/or anatomical environment. For example, there may be particles or clusters, which tend to adhere to each other in blood vessels, but may exhibit a different behavior in other biological environments. In particular, the clustering type may be defined based on dose emission characteristics of a radioembolization particle micro cluster. Thus, radioembolization particle micro clusters belonging to a similar cluster type may show similar dose emission characteristics. In particular, the clustering type may be defined based on any combination of the aforementioned examples. In particular, the clustering type may provide a framework for grouping embolization particle micro clusters into different groups.

The clustering type determination algorithm may be or may include a machine learning algorithm. In general, a machine learning algorithm mimics cognitive functions that humans associate with other human minds. In particular, by training based on training data, the machine learning algorithm is able to adapt to new circumstances and to detect and extrapolate patterns. Other terms for machine learning algorithm may be machine-learned function, trained function, trained machine learning model, trained mapping specification, mapping specification with trained parameters, function with trained parameters, algorithm based on artificial intelligence, or machine learned algorithm.

In general, parameters of a machine learning algorithm can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning algorithm can be adapted iteratively by several steps of training.

In particular, a machine learning algorithm can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network. In particular, the machine learning algorithm may be trained for convolutional pattern recognition, and may be specifically training on medical images.

For instance, the machine learning algorithm may be configured to extract one or more features from the input data, in particular from the medical image and/or the particle density map, and map/classify these features into a feature space associated with different cluster properties, such as cluster dimensions, cluster shapes, and/or clustering types, of radioembolization particle micro clusters.

The usage of machine learning algorithms in general has the advantage that a more comprehensive and faster screening of the available information can be made. In this regard, machine learning algorithms may identify patterns and attributes in the available data that are not accessible for a human.

In particular, the machine learning algorithm may be trained to distinguish between clustering types based on medical images and/or particle density maps. In particular, the machine learning algorithm may be trained to classify clustering types based on medical images and/or particle density maps. In particular, the machine learning algorithm may be trained to segment specific radioembolization particle micro clusters based on medical images and/or particle density maps. In particular, the machine learning algorithm may be trained to identify clustering properties of radioembolization particle micro clusters based on medical images and/or particle density maps.

The machine learning algorithm may be trained to further take into account anatomical structures and tissue in the target area, when determining a clustering type, segmenting micro clusters, classifying micro clusters etc.

In the training phase of the machine learning algorithm, for a certain anatomical structure, in particular for specific vessel arrangements, and/or specific tissue surroundings, the clustering behavior of radioembolization particles may be studied via numerical simulations, ex-vivo tissue studies, vessel flow phantoms or other approaches. Similar approaches as for determining and/or parametrizing the above-explained saturation curves may be applied. The imaging characteristics of particular clusters found may be measured and/or simulated.

The found clusters may be grouped into a number of clustering types, in particular based on the measured and/or simulated imaging characteristics. Simulations of the self-absorption behavior and/or the cross-absorption behavior may be conducted for the clustering types, in particular Monte-Carlo simulation may be conducted. The determined clustering types and corresponding medical images may be used to train the machine learning algorithm, e.g. deep neural network, to distinguish/detect/segment different clustering types from medical images, in particular from CBCT/CT/MRI image data.

Thus, in a preferred embodiment, the machine learning is trained to classify a micro cluster of radioembolization particles into one of a plurality of pre-defined clustering types wherein the plurality of pre-defined clustering types is pre-defined based on the dose emission characteristics of a cluster of radioembolization particles and/or based on the imaging characteristics of a cluster of radioembolization particles and/or based on the clustering properties of a cluster of radioembolization particles.

Preferably, the imaging characteristics and/or dose emission characteristics have been determined for each clustering type by means of Monte-Carlo-Simulations and/or experiments, as explained above. Similar to the determination of the saturation curves, the determination of clustering types may be executed before the execution of the claimed method or as part of the claimed method.

Thus, using a machine learning algorithm to determine and/or classify clustering types and/or to segment clusters and/or other relevant clustering information, allows for an efficient determination of effective dose applied. At run time, the particle distribution information, such as a particle density density map may be loaded, and different clustering types may be recognized in the image. This allows for specific clustering-type-specific particle-density-to-dose translation curves locally across the image. This means that local differences in self-absorption can be accurately reflected.

Preferably, the method includes a step of determining an anatomical information of an anatomical structure in the target area. In particular, the anatomical structure may be a vessel. In particular, the anatomical information may be determined by means of inputting the medical image to a machine learning algorithm. In particular, the machine learning algorithm may be trained to segment the anatomical structure, e.g. by means of an appropriate segmentation algorithm. In particular, the machine learning algorithm may be trained to classify the anatomical structure as belonging to a pre-defined category, for example, to a pre-defined type of vessel.

The anatomical information may be or may include the size of the anatomical structure, the dimensions of the anatomical structure, the shape of the anatomical structure, the branching of the anatomical structure, the location of the anatomical structure and/or the type of the anatomical structure. The anatomical information may include a classification of the anatomical structure.

Preferably, the step of determining particle distribution information and/or the step of determining an effective dose emitted by the radioembolization particles and/or by a micro cluster of radioembolization particles is further based on the anatomical information of the anatomical structure.

In an exemplary embodiment, in particular, before the anatomical information is determined, a particle density map may be combined and/or fused with a medical image, to enable a better identification of anatomical structures, in particular vessels, which shall be analyzed by means of the machine learning algorithm, in particular segmented. The medical image may be the same medical image as used for determining particle distribution information or the medical image may be an additional medical image, which can be loaded and fused with the particle density map. In particular, images from the same and/or other imaging modalities may be used. For example, pre-SIRT mappings of anatomical structures like vessels and/or CT images with contrasted vasculature may be suitable. The anatomical structures may be segmented from these fused images and/or from other medical images and/or particle density maps. This way, the machine learning algorithm can also take into account topology, size and branching of anatomical structures in spatial relation to the particle distribution and/or the identified particle clusters. Therefore, the identification and segmentation of micro clusters is more precise. Thus, also the determination of effective dose is more precise.

According to an aspect, a data processing apparatus comprising means for carrying out the steps of the method according to any of the claimed embodiments is provided.

According to an aspect, a computer program product is provided, wherein the computer program product comprises instructions, which, when the program is executed by a computer, cause the computer to carry out any of the above-mentioned embodiments.

The computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to an aspect, a computer-readable storage medium is provided, comprising instructions, which, when executed by a computer, cause the computer to carry out the steps of the method according to any of the above-mentioned embodiments.

According to an aspect, the invention further relates to a system for determining an effective dose emitted by radioembolization particles. The system includes a medical imaging system, in particular a CBCT, CT or MRI imaging system. The system further includes a data processing unit, capable of executing the method according to any of the previously presented embodiments. Thus, injected radiopaque and/or MRI-imageable radioembolization particles, e.g., SIRT beads, which emit nuclear radiation to surrounding material/tissue of an examination object or patient, can be detected and the effective dose emitted by the radioembolization particles can be effectively and correctly determined.

All in all the presented methods and systems enable more precise determination and measurements of dosimetry, in particular of the emitted effective dose of micro clusters of radioembolization particles. The self-shielding and/or cross-shielding of nuclear emission of radioembolization particles, in particular of Y90/SIRT beads, is taken into account. For internal radiation therapy treatment, the presented approach even outperforms SPECT/CT dosimetry in estimating the local biological effect of the treatment. The presented methods and systems enable a more accurate determination of dose emitted by radioembolization particles.

The embodiments and features described with reference to the method of the present invention apply mutatis mutandis to the device and/or the system of the present invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
- Fig. 1: schematically shows an embodiment of a system for determining an effective dose;
- Fig. 2: shows a schematical drawing of anatomical structure with a micro cluster of radioembolization particles; and
- Fig. 3: shows a schematical illustration of a method for determining an effective dose.

In the Figures, like reference numerals designate like or functionally equivalent elements, unless otherwise indicated. To improve the understandability of the present specification some of the described examples are only explained in the context of radiopaque radioembolization particles and CT imaging. However, the invention is also applicable to other types of embolization particles and/or to other types of imaging.

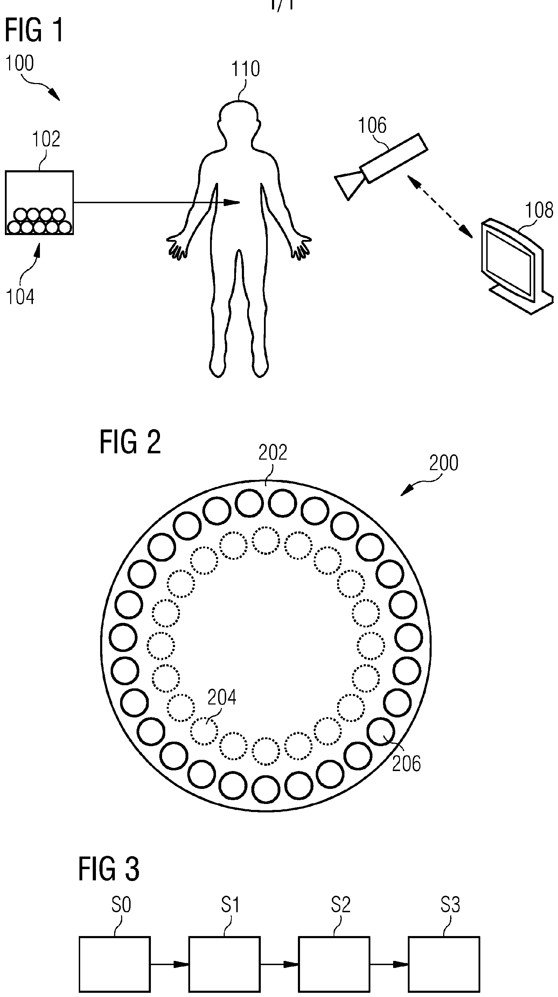

Referring now to Figure 1, an example radioembolization system 100 is shown. The radioembolization system 100 may include a source 102 of the radioembolization particles 104, an imaging device 106, and a computing device 108. The source 102 of radioembolization particles 104 may be any suitable receptacle such as a bag, syringe, or other container that can hold the radioembolization particles 104 for delivery to a patient 110. The radioembolization particles 104 may be delivered into the bloodstream of the patient 110 using a catheter or other suitable device. The source 102 may be a syringe that can be injected with a saline or other delivery fluid.

The radioembolization particles 104 may be delivered into a predetermined target area, in particular an anatomical structure, in particular blood vessels and/or vascular network leading to a target tissue, e.g., a tumor. For example, if the radioembolization treatment is for treatment of a tumor in the liver, the liver may be imaged prior to the radioembolization treatment to determine the anatomical structures, in particular the vasculature, which delivers blood to the tumor. During the radioembolization treatment, the catheter may be positioned to deliver the radioembolization particles to this predetermined vasculature.

The radioembolization particles 104 of the present disclosure, may be both radiopaque and radioactive when delivered to the target tissue of the patient 110. The imaging device 106 can be used to obtain an image of the radioembolization particles 104 in the patient 110. Due to the radiopaque characteristics of the embolization particles 104, the location and distribution of the radioembolization particles 104 can be seen in the captured image. In the presented embodiment, the imaging device 106 is a portable, x-ray-based device that can be used in the operating room or other clinical setting in which the radioembolization treatment is being performed. The imaging device 106 may be, for example, a portable x-ray device or a cone-beam CT imaging device. Such devices may be used, traditionally, to view a location of a catheter, needle or other medical device relative to the target tissue. The radiopaque radioembolization particles of the present disclosure are also visible in the images captured by the imaging device 106.

The images obtained by the imaging device 106 may be provided to the computing device 108. The images acquired prior to the radioembolization treatment as well as the images acquired during or after the treatment may be delivered to the computing device 108. The images can be displayed or analyzed by suitable dose mapping engines or other software to determine a dose map that describes the radiation dose delivered to the target based on the location and distribution of the visible radiopaque radioembolization particles.

If the medical professional and/or the computing device 108 determines that the effective radiation dose is insufficient and/or if the location and distribution of the radioembolization particles is unsatisfactory for the desired treatment, the distribution and/or location of the radioembolization can be changed and/or supplemented. Additional quantities of radioembolization particles can be delivered to the target area, for example. Such changes can be made to deliver satisfactory radiation doses to the target tissue and/or to prevent undesired damage to healthy tissue.

In some embodiments of the present disclosure, the radioembolization particles 104 are glass radioembolization particles. Such particles can be made in various sizes. In some examples, the glass radioembolization particles may be generally spherical in shape and may have a diameter of about 20 to about 30 micrometers in diameter. Other suitable sizes can also be used.

The glass radioembolization particles may be made of various suitable materials. In some examples, the glass radioembolization particles are made of a composition of Yttrium and Silicon. The glass radioembolization particles are biocompatible to be delivered into a target tissue of a patient.

Figure 2 schematically depicts a drawing of an anatomical structure 202 with a micro cluster 200 of radioembolization particles 204 and 206, which shall emit radiation to tissue surrounding the anatomical structure 202.

In the schematical illustration of Figure 2, only two circles of radioembolization particles 204, 206 are depicted to facilitate the understanding of the concept. Clearly, particle micro clusters 200 may comprise more complicated shapes, structures and more particles. However, the concept, which is explained in the following by means of the schematical illustration is also applicable to more complex clustering structures.

The inner radioembolization particles 204 are surrounded by outer radioembolization particles 206. The radiation emitted by the outer radioembolization particles 206 is directly emitted to the surrounding tissue without being intercepted or shielded by other particles. Therefore, the radiation of the outer particles 206 fully contributes to the effective dose emitted by the particle cluster 200. By contrast, the radiation of the inner particles 204 has to pass through the outer particle layer 206 before having an effect to the surrounding tissue.

When passing the outer particles 206, the radiation originating from the inner particles 204 is at least partially shielded by the outer particles 206, in particular due to the radiopaque characteristics of the particles 206. Thus, not all of the emitted radiation passes outside of the anatomical structure 202, but a fraction of the radiation emitted by the inner particles 204 is absorbed due to the self-shielding and/or cross-shielding of the particle cluster 200. In consequence, the absorbed radiation does not contribute to the effective emitted dose of the particle cluster 200.

A proportional mapping between a particle distribution, in particular a particle density in the anatomical structure, and the effective dose emitted by the particle cluster does not take into account this self-shielding effect. Therefore, there is a need for a better and more precise estimation and/or determination of the effective dose emitted by a particle cluster 200.

Figure 3 shows a schematical illustration of a method for determining an effective dose. The method may be executed by a computing device or computing unit, in particular the computing unit as shown in the context of Figure 1.

The method may describe a step S1 of obtaining a medical image. The image may include a target area with a plurality of image-detectable radioembolization particles.

In a step S2, an image analysis of the medical image may be conducted to determine particle distribution information.

In a step S3 an effective dose emitted by the radioembolization particles may be determined. For this, a non-linear or non-proportional distribution-to-dose mapping, in particular a sub-linear or sub-proportional distribution-to-dose mapping, may be applied to the determined particle distribution information.

In one embodiment, the distribution-to-dose mapping may include a sub-proportional relationship between a particle density of a determined micro cluster and an effective dose emitted by the micro cluster.

In another embodiment, the distribution-to-dose mapping may include a plurality of clustering-type specific correlation functions/translation curves, each representing a specific correlation between particle distribution information and an effective dose, wherein the correlation is specific for a particular type of particle cluster, e.g. for a specific 3D-shape of particle cluster, and/or a specific 3D distribution of particle density in a particle cluster.

The clustering type of a particle cluster may be determined by means of a machine learning algorithm. The training of the machine learning algorithm may be executed in a step S0 before executing the method or as a part of the method.

To gather information about the clustering behavior of several algorithms, numerical simulations and/or ex-vivo tissue studies may be conducted and/or vessel flow phantoms may be investigated. Here, certain, exemplary vessel arrangements and/or tissue surroundings may be identified and investigated.

The imaging characteristics of different exemplary particle clusters may be investigated, e.g. by measurements or by simulations. Based on the imaging characteristics, groups of particle clusters with similar imaging characteristics may be identified. As the underlying structure of the particle clusters belonging to a group is known, the self-shielding behavior of the particle clusters can be measured or simulated, e.g., by means of Monte-Carlo simulations of the single particle clusters of a group. The results, describing the self-shielding behavior of the single particles clusters in a group may be combined to receive an overall self-shielding behavior of the particle cluster group, e.g., by means of statistical measures. The overall self-shielding behavior may be an overall distribution-to-dose mapping, describing the relation between particle distribution information and emitted effective dose for an entire particle cluster group.

An artificial intelligence algorithm, also known as machine learning algorithm, may be trained to distinguish between the respective groups of particle clusters based on the imaging characteristics identified in the context with the above-explained process. In particular, the artificial intelligence algorithm may be trained to distinguish and/or detect and/or segment and/or classify different clustering types. Optionally anatomical structures in the target area, e.g., vessel structures may be taken into account, when determining a clustering type of a particular radioembolization particle cluster in a target area.

At run time, in particular in step S3, the particle distribution information, in particular a particle density map, may be input the machine learning algorithm and the machine learning algorithm may detect and/or determine different clustering types of radioembolization particle clusters in one or more target areas of the medical image.

Depending on the identified clustering type, a corresponding distribution-to-dose translation curve is selected, and applied locally to determine the effective dose for a particular radioembolization particle cluster in the medical image. Thus, for each particle cluster in the medical image, a suitable translation curve can be selected, wherein the selected translation curve may differ from the translation curves selected for other particle clusters. In consequence, local differences in self-absorption can be accurately reflected. The determination of effective dose values is more precise.

Optionally, other medical images, such as pre-SIRT images or CT images conducted under administration of contrast agent, showing the anatomical structures of the patient, may be obtained. The anatomical structures may be segmented in these images. For this, the other medical images may be taken as stand alone and/or the other medical images may be fused with the medical image and/or a particle density map generated from the medical image. This way, the machine learning algorithm can also take into account topology, size and/or branching behavior of the anatomical structures. This information can in particular be put in context with the particle distribution information, in particular with the particle density. The segmentation/detection of particle clustering types and/or the selection of appropriate translation curves may be based on that context information.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims. Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein. While various aspects and embodiments have been disclosed, other aspects and embodiments are contemplated. The various aspects and embodiments disclosed are for purposes of illustration and are not intended to be limiting, with the true scope and spirit being indicated by the following claims.

## Claims

1. Computer-implemented method for determining an effective dose emitted by radioembolization particles, the method comprising:
- obtaining a medical image including at least one target area of a patient, the target area including a plurality of radioembolization particles, wherein at least a part of the radioembolization particles is image-detectable;
- determining, by means of an image analysis of the medical image, at least one particle distribution information, the particle distribution information indicating a distribution of the radioembolization particles in the target area;
- determining an effective dose emitted by the radioembolization particles, by applying a non-proportional distribution-to-dose mapping to the determined at least one particle distribution information.

2. Method according to claim 1, wherein the radioembolization particles are at least partially radiopaque and/or detectable in an MRI image.

3. Method according to any of the preceding claims, wherein the radioembolization particles include radiopaque material without radioactive properties.

4. Method according to any of the preceding claims, wherein the medical image includes a plurality of target areas, and the method comprises:
- determining particle distribution information for each of the target areas; and
- determining an effective dose emitted by the radioembolization particles, by applying a non-proportional distribution-to-dose mapping to the determined respective particle distribution information.

5. Method according to any of the preceding claims, wherein the step of determining at least one particle distribution information includes:
- determining, based on the medical image, a particle density map, wherein a value of image points of the particle density map correlates with the density of radioembolization particles in the corresponding image points of the medical image.

6. Method according to any of the preceding claims, wherein the non-proportional distribution-to-dose mapping is based on the dose self-shielding and/or the dose cross-shielding between the radioembolization particles.

7. Method according to claim 6, wherein the particle distribution information includes an average density of the radioembolization particles in the target area, and wherein the distribution-to-dose mapping includes a non-proportional, in particular sub-proportional, saturation curve, which is indicative of a correlation between the average particle density and the effective dose.

8. Method according to claim 6 or 7, wherein the particle distribution information includes the local distribution of radioembolization particles in the target area, in particular a shape and/or distribution of radioembolization particle micro clusters, in particular a 3D shape and/or 3D distribution of radioembolization particle micro clusters.

9. Method according to claim 8, wherein a clustering type of an radioembolization particle micro cluster is determined by inputting the medical image and/or the particle density map into a machine learning algorithm, wherein the machine learning algorithm is trained to determine a clustering type, in particular based on imaging characteristics of the radioembolization particle micro clusters.

10. Method according to claim 9, wherein the machine learning algorithm is trained to classify a micro cluster of radioembolization particles into one of a plurality of pre-defined clustering types, wherein, in particular, the plurality of pre-defined clustering types are pre-defined based on the dose emission characteristics of a cluster of radioembolization particles.

11. Method according to any of claims 9 or 10, wherein imaging characteristics and/or dose emission characteristics have been determined for each clustering type by means of Monte-Carlo-Simulations and/or experiments.

12. Method according to any of the preceding claims, wherein the method includes:
- determining anatomical information of an anatomical structure in the target area, in particular a vessel, by means of segmenting the anatomical structure, wherein the step of determining particle distribution information and/or the step of determining an effective dose emitted by the radioembolization particles is further based on the anatomical information of the anatomical structure.

13. A data processing apparatus comprising means for carrying out the steps of the method of any of claims 1 to 12.

14. A computer program product comprising instructions, which, when the program is executed by a computer, cause the computer to carry out the steps of the method of any of claims 1 to 12.

15. A computer-readable storage medium comprising instructions, which, when executed by a computer, cause the computer to carry out the steps of the method of any of claims 1 to 12.
